**Europäisches Patentamt**

**European Patent Office**

**Office européen des brevets**

(11) Veröffentlichungsnummer: **0 188 701**
A2

## (12) EUROPÄISCHE PATENTANMELDUNG

(21) Anmeldenummer: 85115123.3

(22) Anmeldetag: 28.11.85

(51) Int. Cl.⁴: **A 61 B 17/39**

(30) Priorität: 22.01.85 DE 3501863

(43) Veröffentlichungstag der Anmeldung: 30.07.86
Patentblatt 86/31

(84) Benannte Vertragsstaaten: AT CH DE FR GB IT LI

(71) Anmelder: **Sutter, Hermann, Steinmatten 28,
D-7803 Gundelfingen (DE)**

(72) Erfinder: **Sutter, Hermann, Steinmatten 28,
D-7803 Gundelfingen (DE)**

(74) Vertreter: **Patentanwälte Dipl.-Ing. Hans Schmitt
Dipl.-Ing. Wolfgang Maucher, Dreikönigstrasse 13,
D-7800 Freiburg I.Br. (DE)**

(54) **Bipolares Koagulationsinstrument.**

(57) Ein bipolares Koagulationsinstrument (1) mit zwei Greif- oder Klemmschenkeln (2), die als verschiedene Pole bildende Koagulationselektroden dienen, an den Stellen dauernder Berührung bzw. gegenseitiger Befestigung gegeneinander isoliert sind und zum Verbinden mit einer Hochfrequenzquelle an dem ihren Arbeitsenden abgewandten Ende ausgebildet sind und ein die elektrischen Leitungen (5) von der Hochfrequenzquelle aufweisendes Verbindungsstücke (6) zum Herstellen des Kontaktes mit den Elektroden bzw. Schenkeln (2) sind zum Zusammenführen und gegenseitigen Berühren für die Überleitung der Hochfrequenzenergie auf das Instrument (1) ausgebildet. Dabei sind die Kontakte (7) der Elektroden (2) und die Kontakte (8) des Verbindungsstückes (6) als durch den Benutzer in Berührposition zusammenzuhaltende Berührkontakte ausgebildet. Sie sind also nicht formschlüssig oder klemmend verbindbar. Somit kann mit dem Instrument (1) zunächst ohne Behinderung durch Stromanschlußkabel (5) gearbeitet und dann in einer bestimmten Halteposition durch Berührung mit dem Verbindungsstück (6) bipolar koaguliert werden. Besonders günstig ist es, wenn die Kontakte (7) des Instrumentes (1) als kugeliger Vorsprung und die Kontakte (8) als Kontaktfedern in einem konischen Hohlraum (9) angeordnet sind.

PATENTANWÄLTE

DIPL.-ING. H. SCHMITT

DIPL.-ING. W. MAUCHER

Herr
Hermann Sutter
Steinmatten 28
7803 Gundelfingen

78 FREIBURG I. BR.
DREIKÖNIGSTR. 13
TELEFON: (0761) 20673
70774

MR/bö

UNSERE AKTE · BITTE STETS ANGEBEN!

E 85 491 MR

## Bipolares Koagulationsinstrument

Die Erfindung betrifft ein bipolares Koagulationsinstrument mit zwei Greif- oder Klemmschenkeln, die als verschiedene Pole bildende Koagulationselektroden dienen, zumindest an Stellen dauernder gegenseitiger Berührung gegeneinander isoliert sind und zum Verbinden mit einer Hochfrequenzquelle an dem ihren Arbeitsenden abgewandten Ende ausgebildet sind, wobei ein die elektrischen Leitungen von der Hochfrequenzquelle aufweisendes Verbindungsstück zum Herstellen des Kontaktes mit den Elektroden vorgesehen ist.

Ein solches bipolares Koagulationsinstrument ist als Pinzette aus der DE-PS 30 12 849 insbesondere für die Mikro-Chirurgie bekannt. Die beiden Pinzettenschenkel sind dabei als Greifschenkel ausgebildet und in ihrem gegenseitigen Befestigungsbereich gegeneinander isoliert. Sie sind über die Befestigungsstelle nach hinten verlängert und bilden so Stromanschlußvorsprünge, auf die ein die Stromanschlußkabel bzw. elektrischen Leitungen von der Hochfrequenzquelle aufweisender Stecker als Verbindungsstück aufgesteckt werden kann, so lange mit diesem Instrument gearbeitet wird.

In der Allgemeinchirurgie und Orthopädie (Makro-Chirurgie) werden zum Koagulieren und Blutstillen die normalen, im Instrumentensieb vorhandenen anatomischen und chirurgischen Pinzetten verwendet. Mit solchen bekannten Pinzetten wird während der Operation fassend und präparierend gearbeitet. Eine bipolare Koagulation ist dabei mit diesen Instrumenten nicht möglich, so daß auf die monopolare Koagulation mit den bekannten Nachteilen für den Patienten ausgewichen werden muß. Bei der monopolaren Koagulation ergeben sich Gefahren daraus, daß diese monopolare Koagulation unkontrolliert in der Tiefe und seitlich in Richtung Neutralelektrode abläuft, so daß ungewollt in der Nähe der Koagulationsstelle befindliche Nervenbahnen und Blutgefäße angegriffen oder zerstört werden können.

Da Chirurgen zügig arbeiten müssen und dabei daran gewöhnt sind, mit Pinzetten zu arbeiten, die sowohl zum Fassen von Gewebsteilen als auch zum Präparieren und gegebenenfalls zum monopolaren Koagulieren verwendet werden können, sind die bekannten bipolaren Koagulationspinzetten für die Allgemeinchirurgie und Orthopädie bisher nicht oder nur in geringem Maße zum Einsatz gekommen, weil diese ein Anschlußkabel aufweisen. Hat ein Operateur ein blutendes Gefäß mit einer normalen Arbeitspinzette gefaßt und die Blutung zum Stillstand gebracht, müßte er für eine bipolare Koagulation diese Arbeitspinzette gegen eine bipolare Koagulationspinzette mit Anschlußkabel austauschen. Dies würde bedeuten, daß das Gefäß erneut zu bluten beginnt und die Operationswunde durch die Blutung unübersichtlich würde. Als Arbeitspinzetten sind jedoch die bekannten bipolaren Pinzetten mit Anschlußkabel ungeeignet, weil das Kabel beim Arbeiten störend und hinderlich wäre. Vergleichbares gilt auch für chirurgische Klemmen.

Es besteht deshalb die Aufgabe, ein bipolares Koagulationsinstrument der eingangs erwähnten Art zu schaffen, mit welchem fassend, haltend und/oder präparierend gearbeitet werden kann, ohne daß das für die Koagulation erforderliche Kabel stört, mit dem aber dennoch im Bedarfsfall eine bipolare Koagulation ohne umständliche Manipulationen möglich ist.

Zur Lösung dieser Aufgabe ist das eingangs genannte bipolare Koagulationsinstrument dadurch gekennzeichnet, daß die Kontakte der Elektroden und die des Verbindungsstückes als durch den Benutzer in Berührposition zusammenzubringende Berührkontakte ausgebildet sind. Die Kontakte sind also nicht formschlüssig oder klemmend verbindbare Kontakte mit entsprechender Kupplung der Kontakte, wie sie bei der bipolaren Koagulationspinzette gemäße DE-PS 30 12 849 zwischen den Stromanschlußvorsprüngen und dem Stecker vorgesehen sind, ein echter Stecker vermieden ist.

Dadurch ist es möglich, zunächst einmal mit dem bipolaren Koagulationsinstrument in üblicher Weise fassend oder präparierend oder auch haltend und klemmend zu arbeiten und dann durch die entsprechende Berührung mit dem die elektrische Leitung aufweisenden Verbindungsstück zu koagulieren. Eine einfache Berührung der Kontakte beispielsweise durch eine Hilfsperson stört dabei die jeweils eingenommene Position des Instrumentes und den Operateur nicht, während ein Aufstecken eines Klemmsteckers in dieser Position zu unkontrollierten und ungewollten Bewegungen der Arbeitsspitzen führen würde. In scheinbar widersprüchlicher Weise wird somit ein bipolares Koagulationsinstrument zur Verfügung gestellt, welches zunächst ohne störende Kabel und elektrische Leitungen gehandhabt werden kann, in einer einmal gewählten Position dann aber aufgrund der vorhandenen Kontakte und deren entsprechende Berührung zum bipolaren Koagulieren verwendet werden kann.

Für eine gute Handhabung und Bedienbarkeit insbesondere beim Koagulieren ist es vorteilhaft, wenn die Kontakte des einen gegen das andere zusammenzuführende Teil in einem Hohlraum und die Kontakte des anderen Teiles an einem Vorsprung, der in den Hohlraum paßt, vorgesehen sind. Dadurch erhalten die gegensätzlichen Pole beim Zusammenführen in Berührposition eine ausreichende Führung, um eine schnelle Koagulation zu ermöglichen.

Der Kontakthohlraum ist zweckmäßigerweise im Querschnitt kreisförmig und enthält wenigstens zwei einander vorzugsweise gegenüberliegende Kontaktfedern od. dgl. Kontaktflächen. Der Kontaktvorsprung des anderen Teiles ist im Querschnitt zweckmäßigerweise ebenfalls kreisförmig, wobei die Isolierung auf einem Durchmesser liegt. Durch Aufstülpen des Kontakthohlraumes auf den Kontaktvorsprung oder Einführen des Kontaktvorsprunges in den Kontakthohlraum wird also sehr schnell eine entsprechende Kontaktgabe erzielt. Sollten dabei zufällig beide Kontaktflächen auf die Isolierung des Kontaktvorsprunges treffen, genügt eine geringfügige Verdrehung, um die Koagulation in Gang zu setzen.

Zweckmäßig ist es, wenn die Isolierung der Pole des Kontaktvorsprunges in Längsrichtung des Zwischenraumes zwischen den Schenkeln bzw. in Fortsetzung von deren Isolierung angeordnet ist, vorzugsweise einstückig mit dieser ist. Ein einziges Isolierstück kann dann sowohl die Instrumentenschenkel als auch die Pole im Berührbereich gegeneinander isolieren.

Der Kontakthohlraum des einen Teiles kann als Hohlkonus

5

ausgebildet sein, der sich von seiner Öffnung nach innen verengt und in dessen Wandbereich die beiden einander gegenüberliegenden Kontaktfedern od. dgl. Kontaktstreifen angeordnet sind. Dadurch ist das Zusammenführen der beiden zu berührenden Teile vereinfacht, weil ein Konus mit einer weiten Öffnung gut über den Vorsprung gestülpt werden kann, bei weiterer Bewegung dann aber bald in den gewünschten Berührkontakt übergeht.

Der Kontaktvorsprung des anderen Teiles kann ein Kegel oder Kegelstumpf sein, der zu dem Hohlkonus paßt.

Besonders zweckmäßig ist es aber, wenn der Vorsprung des anderen Teiles kugelförmig ist, wobei die Kugel auf einem vorzugsweise im Querschnitt kreisförmigen Hals oder Stiel angeordnet sein kann. Dies ermöglicht es nämlich, den den Hohlkonus aufweisenden Teil auch unter einem gewissen Winkel zusammenzuführen, was bei einer Operation, bei welcher eine Hilfsperson den Berührkontakt zwischen dem die elektrischen Leitungen von der Hochfrequenzquelle aufweisenden Verbindungsstück und dem Instrument herstellen soll, von erheblichem Vorteil ist.

Besonders zweckmäßig ist es, wenn der Kontaktvorsprung am den Arbeitsspitzen abgewandten Ende des Instrumentes und der Hohlkonus an einem die Elektrokabel aufweisenden Kontaktstift od. dgl. angeordnet ist. Somit kann dieser Stift mit seinem Hohlkonus auf den Vorsprung des Instrumentes gestülpt werden, wenn eine Koagulation durchgeführt werden soll. Die Ausbildung von Kugel und Konus erleichtert dabei das Berühren und auch das Halten in Berührposition, weil sich die auf diese Weise aufeinander abgestimmten Teile gut zusammenführen und zusammenhalten lassen. Es braucht lediglich ein gewisser Druck in Längsrichtung des Hohlkonus ausgeübt zu werden.

Eine zweckmäßige Ausführungsform der Erfindung kann dabei darin bestehen, daß die beiden Schenkel, die mit unterschiedlichen Polen verbindbar und gegeneinander isoliert sind, jeweils eine Hälfte des Kontaktvorsprunges aufweisen. Dazwischen befindet sich die schon erwähnte Isolierung. Der Haltevorsprung kann dabei etwa in gerader Verlängerung der Schenkel mit diesen verbunden sein.

Um in weitgehend beliebiger Richtung und Orientierung den Hohlkonus auf den Vorsprung aufstülpen und andrücken zu können und dabei einen Koagulations-Strom zu übertragen, ohne einen Kurzschluß zu erzeugen, ist es besonders zweckmäßig, wenn die Kontaktfedern bzw. Kontaktflächen des Kontakthohlraumes gleich breit oder - bevorzugt - schmaler als die Dicke der Isolierung des Kontaktvorsprunges an dessen Außenseite ist. Dadurch kann verhindert werden, daß eine Kontaktfläche die Isolierung überbrücken könnte, falls diese keine ballige Form hat. Es würde dann nur in einer einzigen Position, in der zufällig die Kontaktfedern genau auf der Isolierung liegen, keine Koagulation stattfinden. Wie schon obenstehend erwähnt, genügt es dann jedoch, das Verbindungsstück geringfügig zu verdrehen, um eine elektrische Verbindung der beiden Pole und damit die Koagulation zu erreichen.

Das als Kontaktstift dienende Verbindungsstück kann zwischen seinem Handgriff und dem den Kontaktkonus aufweisenden Bereich abgewinkelt sein. Dies erleichtert weiter die Handhabung insbesondere, wenn eine seitlich vom Operateur stehende Bedienungsperson dieses Verbindungsstück auf das von dem Operateur gehaltene Instrument aufsetzen soll. Dabei kann der Kontaktstift im Bereich des Handgriffes einen Schalter zum Unterbrechen der Stromzuführ zu dem Kontaktkonus aufweisen. Um für die Benutzer eine größtmögliche Sicherheit zu erzielen, ist es dabei vorteilhaft, wenn der Kontaktkonus in dem Kontaktstift verdeckt und isoliert eingebettet ist.

Vor allem bei Kombination einzelner oder aller vorbeschriebener Maßnahmen und Merkmale ergibt sich ein bipolares Koagulationsinstrument, mit dem vor allem fassend und präparierend und auch klemmend gearbeitet werden kann, ohne dabei durch Stromanschlußkabel behindert zu werden, mit dem aber dennoch in erwünschter und vorteilhafter Weise bipolar koaguliert werden kann, ohne daß dafür ein erheblicher zusätzlicher operativer oder Handhabungs-Aufwand notwendig ist.

Es genügt vielmehr, das entsprechend ausgebildete Instrumentenende mit dem dazu passenden Verbindungsstück zu berühren, um die Koagulation durchzuführen.

Nachstehend ist die Erfindung mit ihren ihr als wesentlich zugehörenden Einzelheiten anhand der Zeichnung noch näher beschrieben. Es zeigt in zum Teil schematisierter Darstellung:

Fig. 1    das Kontaktende einer bipolaren Koagulationspinzette mit einem dazu passenden, in seinem Kontaktbereich teilweise aufgebrochen gezeichneten Verbindungsstück mit Stromanschlußkabeln vor dem Zusammenführen,

Fig. 2    in vergrößertem Maßstab den Kontaktvorsprung des Instrumentes und den Kontakthohlraum des Verbindungsstückes vor dem Zusammenführen und in gestrichelter Darstellung in Berührposition, wobei der Kontaktvorsprung kugelig ausgebildet ist,

Fig. 3    eine der Fig. 2 entsprechende Darstellung, wobei der Kontaktvorsprung kegelförmig ausgebildet ist sowie

Fig. 4 die kugelförmig ausgebildeten Kontakte der Elek-
und 5 trode und die des Verbindungsstückes in zwei verschiedenen Berührpositionen zueinander.

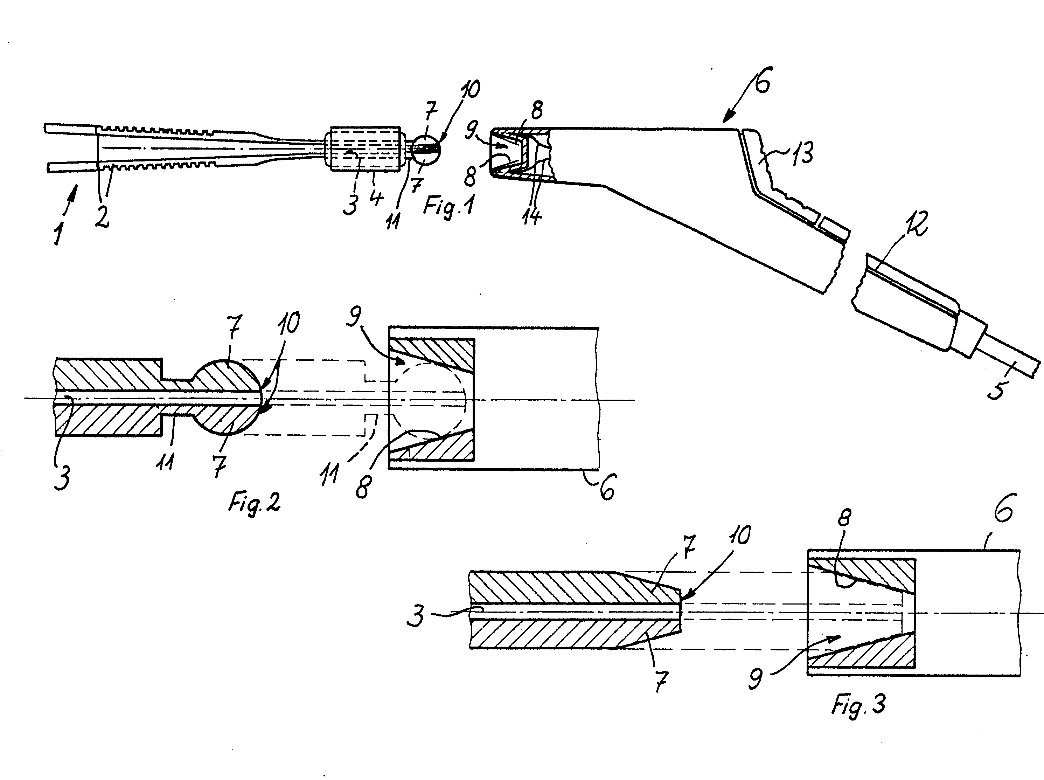

Eine Pinzette 1 ist dadurch als bipolares Koagulationsinstrument ausgebildet, daß ihre beiden Schenkel 2 als verschiedene Pole bildende Koagulationselektroden dienen und
an dem ihren Arbeitsenden abgewandten Ende im Bereich ihrer
gegenseitigen Berührung und Befestigung gegeneinander isoliert sind. Man erkennt im Endbereich der Pinzette 1 in
Fig. 1 schematisiert die Isolierung 3 und eine Haltehülse
4 als Befestigungselement. Ferner erkennt man die Isolierung 3 auch in den übrigen Figuren.

Dieser Endbereich der Pinzette 1 ist ferner zum Verbinden
mit einer Hochfrequenzquelle ausgebildet, wobei ein die
elektrischen Leitungen 5 von der Hochfrequenzquelle aufweisendes Verbindungsstück 6 vorgesehen ist, mit welchem
in noch zu beschreibender Weise der Kontakt zu den Elektroden bzw. Pinzettenschenkeln 2 für den Koagulationsvorgang hergestellt werden kann.

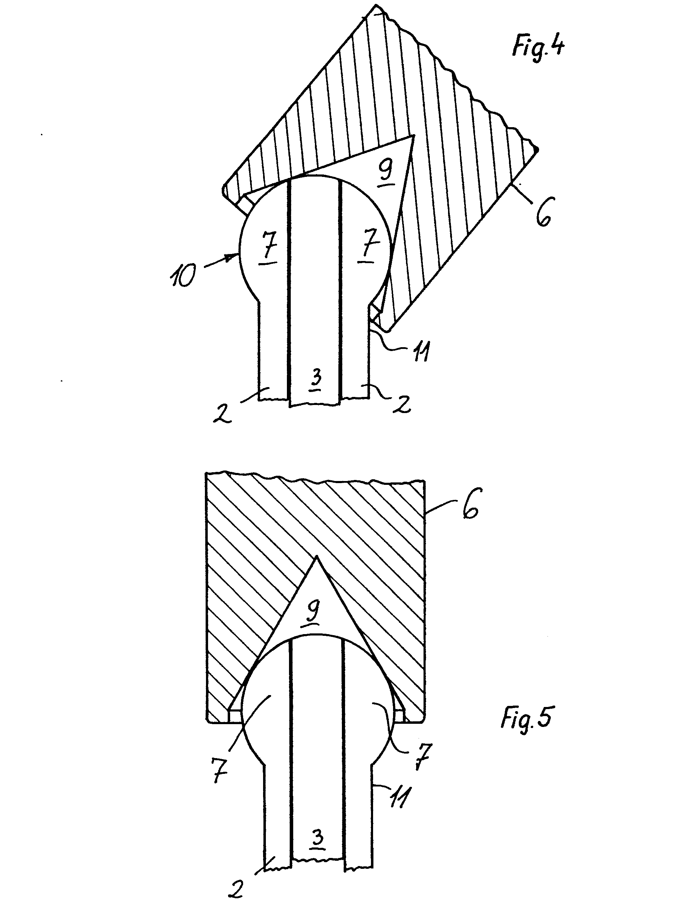

In allen Figuren ist dargestellt, daß die noch näher zu
beschreibenden Kontakte 7 der Elektroden 2 und die Kontakte 8 des Verbindungsstückes 6 als durch den Benutzer
in Berührposition (Fig. 4 und 5 gestrichelte Darstellung
der Fig. 2 und 3) zusammenzuhaltende Berührkontakte ausgebildet sind. Es wird also keine lösbare, aber form- oder
kraftschlüssige oder klemmende Kupplung oder Verbindung
zwischen den Kontakten 7 und 8, sondern lediglich eine
Berührverbindung hergestellt.

0188701

Die Ausbildung der Isolierung 3 und der Befestigung 4 am Ende der Pinzette 1 kann im wesentlichen der des Patentes 30 12 849 oder aber auch anderen derartigen Konstruktionen entsprechen .

Die Kontakte 8 des einen Teiles, im Ausführungsbeispiel des Verbindungsstückes 6, sind dabei in einem Hohlraum 9 und die Kontakte 7 des anderen Teiles an einem Vorsprung 10, der in den Hohlraum 9 paßt, vorgesehen. Dabei sind sowohl der Kontakthohlraum 9 als auch der Kontaktvorsprung 10 im Querschnitt kreisförmig, wobei die Isolierung 3 im Ausführungsbeispiel senkrecht zu den Durchmessern dieses Querschnitts liegt. Die Isolierung 3 der Pole bzw. Elektroden 2 des Kontaktvorsprungs 10 verläuft dabei in Längsrichtung des Zwischenraumes zwischen den Schenkeln 2 bzw. in Fortsetzung von deren Isolierung und ist einstückig mit dieser und hat deshalb auch dasselbe Bezugszeichen 3.

Der Kontakthohlraum 9 ist im Ausführungsbeispiel als Hohlkonus ausgebildet, der sich von seiner Öffnung nach innen verengt und in dessen Wandbereich die beiden einander gegenüberliegenden Kontaktfedern 8 od. dgl. Kontaktstreifen angeordnet sind.

Gemäß Fig. 3 kann der Kontaktvorsprung 10 als Kegel oder Kegelstumpf ausgebildet sein. Gemäß den übrigen Figuren ist er jedoch bevorzugt kugelförmig, wobei die Kugel auf einem vorzugsweise im Querschnitt kreisförmigen Hals 11 oder Stiel angeordnet ist. Dies erlaubt die in den Fig.4 und 5 angedeuteten erheblichen Abweichungen der Orientierung zwischen den beiden in Berührung miteinander zu bringenden Teilen, wobei dennoch in jeder Orientierung Kontakt hergestellt und eine Koagulation durchgeführt werden kann.

10

Im Ausführungsbeispiel ist der Kontaktvorspung 10 am den Arbeitsspitzen abgewandten Ende des Instrumentes 1 und der Hohlkonus 9 an einem die Elektrokabel 5 aufweisenden Kontaktstift, nämlich dem Verbindungsstück 6 angeordnet. Somit kann das Verbindungsstück 6 mit seinem Hohlkonus 9 einfach auf die Kontaktkugel 10 aufgestülpt werden, wobei dieses Aufstülpen unter praktisch jedem Winkel und in praktisch jeder Drehposition zu einem echten Kontakt führt, also eine Koagulation ermöglicht, was die Handhabung vereinfacht und die Arbeitsgeschwindigkeit begünstigt. Beispielsweise kann die Mittelachse des Verbindungstückes 6 gegenüber der des Instrumentes 1 um bis zu 40° nach beiden Seiten abweichen, wie es in Fig. 4 angedeutet ist.

Eine besonders einfache Herstellung des Instrumentes 1 ergibt sich, wenn - wie im Ausführugnsbeispiel - die beiden Schenkel 2, die für den Koagulationsvorgang mit unterschiedlichen Polen verbunden werden und gegeneinander isoliert sind, jeweils eine Hälfte des Kontaktvorsprunges 10 aufweisen bzw. einstückig bilden. Dabei ist der Kontaktvorsprung 10 etwa in gerader Verlängerung der Schenkel 2 mit diesen verbunden, wobei jeder Kontakt 7 mit seinem zugehörigen Instrumentenschenkel 2 einstückig verbunden ist.

Um zu verhindern, daß bei der Berührung eventuell eine Kontaktfeder 8 die Isolierung 3 überbrückt und die beiden Pole 7 gleichzeitig berührt, was zu einem Kurzschluß führen würde, sind die Kontaktfedern 8 bzw. Kontaktflächen des Kontakthohlraumes 9 gleich breit oder bevorzugt schmaler als die Dicke der Isolierung 3 des Kontaktvorsprunges 10 an dessen Außenseite. Somit kann sich allenfalls bei ganz genauer Anlage der Kontaktflächen ausschließlich an der Isolierung 3 ergeben, daß keine Koagulation stattfindet. Eine geringfügige Verdrehung des Verbindungsstückes 6 führt dann aber sogleich zu der gewünschten Kontaktgabe.

In Fig. 1 erkennt man noch, daß das als Kontaktstift dienende Verbindungsstück 6 zwischen einem Handgriff 12 und dem den Kontaktkonus 9 aufweisenden Bereich abgewinkelt ist. Dadurch kann eine seitlich von dem Benutzer der Pinzette 1 stehende Person das Verbindungsstück 6 dennoch bequem und sicher in den gewünschten Berührkontakt mit dem Kontaktvorsprung 10 bringen. Der Kontaktstift 6 hat dabei im Bereich des Handgriffes 12 einen Schalter 13, mit welchem die Stromzufuhr zu dem Kontaktkonus 9 unterbrochen werden kann. Schließlich ist in den Fig. 1 bis 3 angedeutet, daß der Kontaktkonus 9 mit den Zuleitungen 14 zu seinen Kontakten 8 in dem Kontaktstift 6 verdeckt und isoliert eingebettet ist, so daß die Benutzung für alle Beteiligten gefahrlos ist.

Alle in der Beschreibung, der Zusammenfassung, den Ansprüchen und der Zeichnung dargestellten Merkmale und Konstruktionsdetails können sowohl einzeln als auch in beliebiger Kombination miteinander wesentliche Bedeutung haben.

- Ansprüche -

PATENTANWÄLTE

DIPL.-ING. H. SCHMITT
DIPL.-ING. W. MAUCHER

76 FREIBURG i. BR.
DREIKÖNIGSTR. 13
TELEFON: (0761) 10578
70774

12

0188701

Herr
Hermann Sutter
Steinmatten 28
7803 Gundelfingen

MR/bö

UNSERE AKTE · BITTE STETS ANGEBEN!

E 85 491 MR

## Bipolares Koagulationsinstrument

### Ansprüche

1. Bipolares Koagulationsinstrument mit zwei Greif- oder Klemmschenkeln (2), die als verschiedene Pole bildende Koagulationselektroden dienen, zumindest an Stellen dauernder gegenseitiger Berührung und/oder Befestigung gegeneinander isoliert sind und zum Verbinden mit einer Hochfrequenzquelle an dem ihren Arbeitsenden abgewandten Ende ausgebildet sind, wobei ein die elektrischen Leitungen (5) von der Hochfrequenzquelle aufweisendes Verbindungsstück (6) zum Herstellen des Kontaktes mit den Elektroden bzw. Schenkeln (2) vorgesehen ist, d a - d u r c h   g e k e n n z e i c h n e t , daß die Kontakte (7) der Elektroden (2) und  die Kontakte (8) des Verbindungsstückes (6) als durch den Benutzer in Berührposition zusammenzuhaltende Berührkontakte ausgebildet sind.

2. Koagulationsinstrument nach Anspruch 1, dadurch gekennzeichnet, daß die Kontakte (8) des einen gegen das andere zusammenzuführende Teil in einem Hohlraum (9) und die Kontakte (7) des anderen Teiles an einem Vorsprung (10), der in den Hohlraum (9) paßt, vorgesehen sind.

3. Koagulationsinstrument nach Anspruch 1 oder 2, dadurch gekennzeichnet, daß der Kontakthohlraum (9) im Querschnitt kreisförmig ist und wenigstens zwei einander vorzugsweise gegenüberliegende Kontaktfedern od. dgl. Kontaktflächen enthält.

4. Koagulationsinstrument nach einem der Ansprüche 1 bis 3, dadurch gekennzeichnet, daß der Kontaktvorsprung (10) des anderen Teiles im Querschnitt kreisförmig ist, wobei die Isolierung (3) vorzugsweise senkrecht zu einem Durchmesser liegt.

5. Koagulationsinstrument nach einem der Ansprüche 1 bis 4, dadurch gekennzeichnet, daß die Isolierung (3) der Pole des Kontaktvorsprunges (10) in Längsrichtung des Zwischenraumes zwischen den Schenkeln (2) bzw. in Fortsetzung von deren Isolierung angeordnet ist, vorzugsweise einstückig mit dieser ist.

6. Koagulationsinstrument nach einem der Ansprüche 1 bis 5, dadurch gekennzeichnet, daß der Kontakthohlraum (9) des einen Teiles als Hohlkonus ausgebildet ist, der sich von seiner Öffnung nach innen verengt und in dessen Wandbereich die beiden einander gegenüberliegenden Kontaktfedern (8) od. dgl. Kontaktstreifen angeordnet sind.

7. Koagulationsinstrument nach einem der Ansprüche 1 bis 6, dadurch gekennzeichnet, daß der Kontaktvorsprung (10) des anderen Teiles ein Kegel oder Kegelstumpf ist.

8. Koagulationsinstrument nach einem der Ansprüche 1 bis 6, dadurch gekennzeichnet, daß der Vorsprung des anderen Teiles kugelförmig ist, wobei die Kugel auf einem vorzugsweise im Querschnitt kreisförmigen Hals (11) oder Stiel angeordnet ist.

9. Koagulationsinstrument nach einem der vorstehenden Ansprüche, dadurch gekennzeichnet, daß der Kontaktvorsprung (10) am den Arbeitssitzen abgewandten Ende des Instrumentes (1) und der Hohlkonus (9) an einem die Elektrokabel aufweisenden Kontaktstift od. dgl. angeordnet sind.

10. Koagulationsinstrument nach einem der Ansprüche 1 bis 9, dadurch gekennzeichnet, daß die beiden Schenkel (2), die mit unterschiedlichen Polen (7) verbindbar oder verbunden, vorzugsweise einstückig verbunden und gegeneinander isoliert sind, jeweils eine Hälfte des Kontaktvorsprunges (10) aufweisen.

11. Koagulationsinstrument nach einem der Ansprüche 1 bis 10, dadurch gekennzeichnet, daß der Kontaktvorsprung (10) etwa in gerader Verlängerung der Schenkel (2) mit diesen verbunden ist.

12. Koagulationsinstrument nach einem der vorstehenden Ansprüche, dadurch gekennzeichnet, daß die Kontaktfedern (8) bzw. Kontaktflächen des Kontakthohlraumes (9) gleich breit oder schmaler als die Dicke der Isolierung (3) des Kontaktvorsprunges (10) an dessen Außenseite sind.

13. Koagulationsinstrument nach einem der Ansprüche 1 bis 12, dadurch gekennzeichnet, daß das als Kontaktstift dienende Verbindungsstück (6) zwischen seinem Handgriff und dem den Kontaktkonus (9) aufweisenden Bereich abgewinkelt ist.

14. Koagulationsinstrument nach einem der Ansprüche 1 bis 13, dadurch gekennzeichnet, daß der Kontaktstift im Bereich des Handgriffes (12) einen Schalter (13) zum Unterbrechen der Stromzufuhr zu dem Kontaktkonus (9) aufweist.

15. Koagulationsinstrument nach einem der Ansprüche 1 bis 14, dadurch gekennzeichnet, daß der Kontaktkonus (9) mit den Zuleitungen (14) zu seinen Kontakten (8) in dem Kontaktstift (6) verdeckt und isoliert eingetettet ist.

- Zusammenfassung -

Fig. 1

Fig. 2

Fig. 3

1/2

0188701

0188701

Fig.4

Fig.5